# EUROPEAN PATENT APPLICATION

(11) **EP 4 421 081 A1**
(43) Date of publication of application: **28.08.2024**
(21) Application number: 23305231.5
(22) Date of filing: 22.02.2023
(51) Int. Cl.: C07H 7/00, A01P 1/00, A01P 3/00, A01P 5/00, A01P 7/04, A01P 13/00, A01P 21/00, C07H 7/02, A01N 43/08

(54) **ISOLATED 7DSH ANALOGUES, SYNTHESIS AND USES THEREOF**

(71) Applicant: Université de Rennes, 35042 Rennes (FR); École Nationale Supérieure de Chimie, 35700 Rennes (FR); Institut National des Sciences Appliquées de Rennes, 35700 Rennes (FR); Centre national de la recherche scientifique, 75016 Paris (FR); Institut National de la Santé et de la Recherche Médicale, 75013 Paris (FR); Ecole des Hautes Etudes en Santé Publique (EHESP), 35043 Rennes cedex (FR)
(72) Inventor: FERRIERES, Vincent, 35490 GAHARD (FR); SPARFEL, Lydie, 35137 PLEUMELEUC (FR); LAGADIC-GOSSMANN, Dominique, 35440 GUIPEL (FR)
(74) Representative: Novagraaf Technologies

(57) **Abstract**

The present invention belongs to the field of herbicidal composition. It also relates to synthesis and process for the preparation of the biocidal compound having the structure: 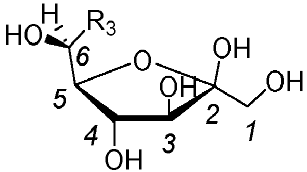 or salts thereof; wherein R₃ one chemical group selected among an ethyl, an isopropyl, a n-butyl, an isobutyl, a cyclopentyl, a cyclohexyl, an allyl or-CH₂-Ph.

## Description

### Technical field

The present invention belongs to the field of biocidal compounds, and more specifically to the synthesis of biocidal compounds, such as herbicidal compounds.

More particularly, the present invention relates to new isolated 7dSh analogues compounds and their use thereof in an herbicidal composition, preferably in method for controlling one or more harmful plants and/or for regulating the growth of one or more plants or for use as an antimicrobial agent. The invention also relates to the method of synthesis of the new isolated 7dSh analogues compounds according to the invention.

In the description below, references between [] refer to the list of references at the end of the examples.

### Technical background

Glyphosate (IUPAC name: N-(phosphonomethyl)glycine) is a broad-spectrum systemic herbicide and crop desiccant. It is an organophosphorus compound, specifically a phosphonate, which acts by inhibiting the plant enzyme 5-enolpyruvylshikimate-3-phosphate synthase. It is used to kill weeds, especially annual broadleaf weeds and grasses that compete with crops. It was discovered to be an herbicide in 1970 was brought to market for agricultural use in 1974 under the trade name Roundup.

Glyphosate was quickly adopted for agricultural weed control. In 2007, glyphosate was the most used herbicide in the United States' agricultural sector and the second-most used in home and garden, government and industry, and commercial applications.

While glyphosate and formulations such as Roundup have been approved by regulatory bodies worldwide, concerns about their effects on humans and the environment persist and have grown as the global usage of glyphosate increases. A number of regulatory and scholarly reviews have evaluated the relative toxicity of glyphosate formulations as an herbicide composition **[1-2]**.

There is therefore a need to find alternative compound to the controversial glyphosate.

7-deoxy-D-altro-2-heptulose or 7dSh is a rare sugar that has recently been reported as a good candidate as a substitute for glyphosate **[3]**. Whether it was produced biotechnologically from Synechococcus elongatus or biocatalytically using S. elongatus transketolase, the quantities extracted are low, and the compound, to the knowledge of Applicants, has never been isolated in exploitable amounts: it remains today as a mixture of compounds comprising various sugars. Also, this method can only lead to one diastereomer, where the carbon in position 6 has a S conformation):

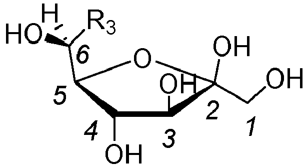

Compound 7dSh was found to have very interesting herbicidal properties and antimicrobial activity.

To date, except for the biocatalytic pathway mentioned above, no synthetic pathway has been identified at the present time for the 7dSh compound or its analogues.

There is therefore a need to develop a process to obtain this compound and its analogues in an isolated form, and in large quantities that allow industrial production and application.

### Definitions

As used herein the terme "biocidal" is defined according to the definition provided by the Directive 98/8/EC of the European Union dated 16 February 1998. Among the biocidal compounds it can be referred to "bactericidal", "virusidal", "pesticidal" such as "insecticidal", "fungicidal" or "herbicidal" compounds.

As used herein, the term "isolated", when applied to the compounds of the present invention, refers to such compounds that are (i) not in a cell or organism and the compounds are separated from at least some components with which they are associated in nature or when they are made, notably by biotechnological (e.g., bacterial or biocatalysis) means and/or (ii) produced, prepared or manufactured by the hand of man. For example, an "isolated" compound refers to a compound isolated from a crude (chemical or biotechnological) reaction mixture following an affirmative act of isolation. The act of isolation involves separating the compound from the other known components of the crude reaction mixture, with some impurities, unknown side products and residual amounts of the other known components of the crude reaction mixture permitted to remain. Purification is an example of an affirmative act of isolation.

As used herein, it is meant by "antimicrobial activity" refers to the ability of a compound to modify a function or metabolic process of a target microorganism, and relates more particularly to inhibition of growth of a microorganism. In aspects of the claimed compound or composition, antimicrobial activity relates to the ability of the compound to kill at least one bacterial species. The term can be manifested as microbiocidal or microbiostatic inhibition of microbial growth. Preferably, as used herein, antimicrobial activity refers to "antibacterial activity" and/or "antifungal activity".

The term "microorganism" herein refers broadly to bacteria, fungi, viruses, and protozoa.

### Detailed description of the invention

The aim of the present invention is to provide a synthetic process for the preparation of 7dSh compound and its analogues that may solve all the problems listed above.

The present invention thus deals with the 7dSh compound and its analogues in isolated form, their synthetic methods or process for their preparation and their applications, such as use in herbicidal composition or as an antimicrobial agent. The isolated compounds according to the invention present the advantage to be easily synthesised, in mild conditions and in yields that allow industrial production and application.

The invention therefore relates to the synthesis of an isolated compound having the structure (V): 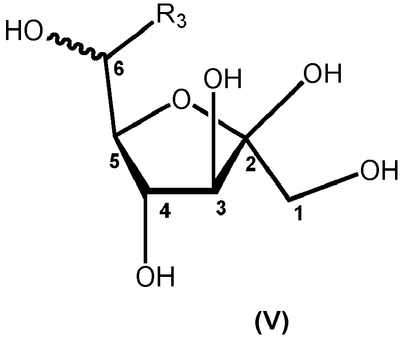 and advantageaously the present invention relates to a process for the preparation of a biocidal compound comprising the steps of:
a) oxidizing primary alcohol group of intermediate (I) wherein R₂ represents a suitable secondary alcohol protective group, such as a benzyl protective group to form intermediate (II) according to the following scheme : 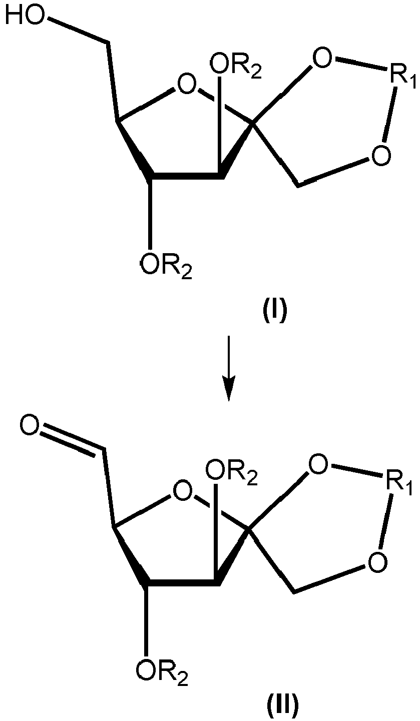 wherein R₂ represents a suitable secondary alcohol protective group, such as a benzyl protective group; and wherein R₁ is selected from saturated and unsaturated linear or branched aliphatic groups, and is preferably selected amongs: alkyl, alkenyl, alkynyl, eventually bearing an aryl group;
b) alkylating the aldehyde moiety of intermediate (II) under suitable conditions to form intermediate (III) : 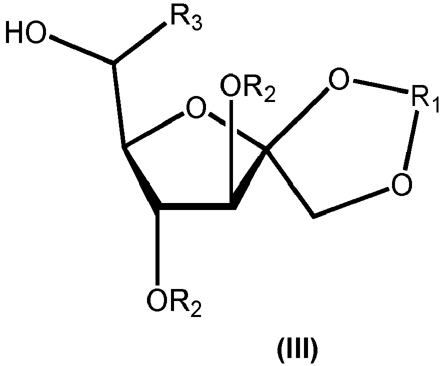 wherein R₃ represents a cyclic or acyclic, substituted or unsubstituted, linear or branched C₁₋₁₂alkyl, C₁₋₁₂alkoxyl, C₁₋₁₀alkyl-CH=CH₂ or -CH₂-Ar group ; and
c) removing the R₂ protective groups to yield a compound having the structure (IV): 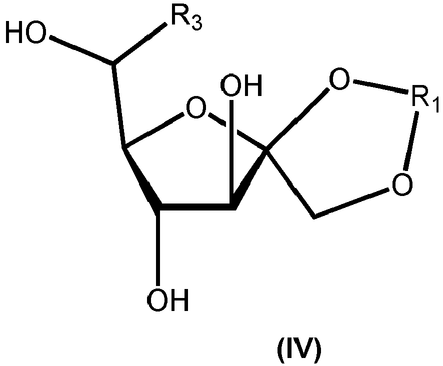
d) removing the R₁ protective groups to yield a compound having the structure (V):

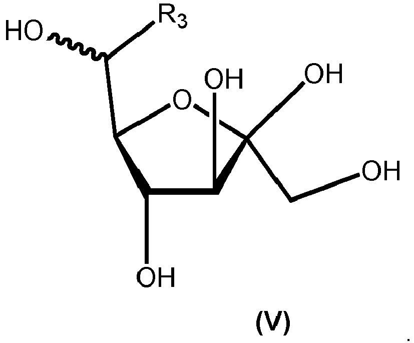

Advantageously, R₂ is a benzyl protective group.

Advantageously, the process according to the present invention comprises a supplementary! step e) performed before step c) and being according to the following:
e) protecting the primary alcool group of intermediate (III) under suitable conditions to form intermediate (VI) : 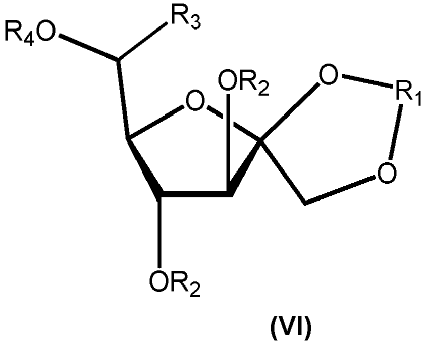 wherein R₄ represents a suitable primary alcohol protective group, such as a benzyl protective group, step c) consisting in deprotecting R₄ together with R₂ protecting group. The inventors have demonstrated in a totally unexpected way that this additional step allows to obtain *in fine* the compound V, in particular when R₃ is a methyl, with a much better degree of purity and keeping a very acceptable global yield.

The compounds (I) is prepared preferably according to the following scheme comprising the steps of:
A1) acetalisation of D-fructose to form an intermediate having the structure : 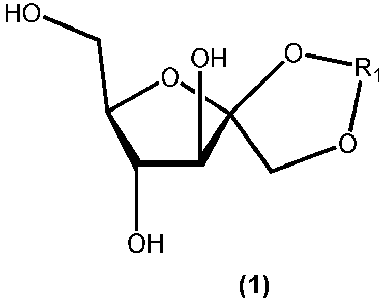 wherein R₁ is selected from saturated and unsaturated linear or branched aliphatic groups, and is preferably selected amongs: alkyl, alkenyl, alkynyl, eventually bearing an aryl group;
A2) protecting the primary OH moiety of intermediate (1) under suitable conditions to form intermediate (2) : 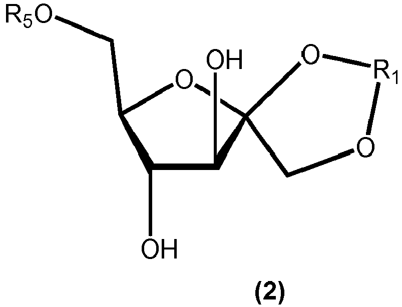 wherein R₅ represents a suitable primary alcohol protective group, such as a silyl ether protective group of structure -SiR₆R₇R₈ wherein R₆, R₇ and R₈ independently represent linear or branched C₁₋₆alkyl;
A3) protecting the secondary OH moieties of intermediate (2) under suitable conditions to form intermediate (3) : 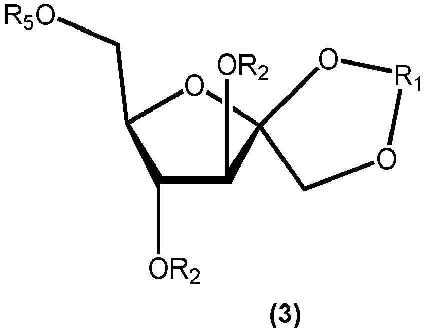 wherein R₂ represents a suitable secondary alcohol protective group, such as a benzyl protective group ;
A4) selectively deprotecting the -OR₅ moiety of intermediate (3) under suitable conditions to form intermediate (I) :

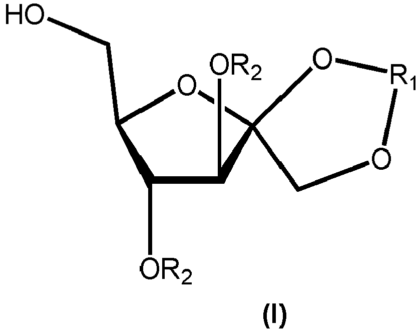

Advantageously, in the process according to the present invention R₂ and R₄ are benzyl protecting groups and step c) consisting of deprotecting the benzyl protective group to provide free -OH groups to form intermediate IV.

Advantageously, in the process according to the present invention R₁ represents one of the following groups selected among:

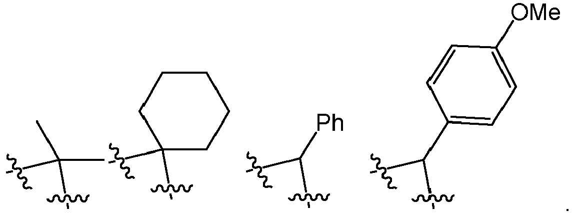

Advantageously, in the process according to the present invention step a) is a mild oxidation step, preferably a Dess Martin oxidation step, which is carried out by reacting intermediate (I) with Dess Martin periodinane.

Advantageously, in the process according to the present invention step b) is carried out by reacting intermediate (II) with R₃Li or (R₃)₂ZnTi(OiPr)₄ ; wherein R₃ represents a cyclic or acyclic, substituted or unsubstituted, linear or branched C₁₋₁₂alkyl, C₁₋₁₂alkoxyl, C₁₋₁₀alkyl-CH=CH₂ or -CH₂-Ph group.

Advantageously, in the process according to the present invention R₃ represents methyl, ethyl, isopropyl, n-butyl, isobutyl, cyclopentyl, cyclohexyl, allyl or-CH₂-Ph; preferably methyl.

The invention also relate to a Isolated compound obtained by the process described within the context of the present invention, and having one of the following formula (V) and (Vbis) : 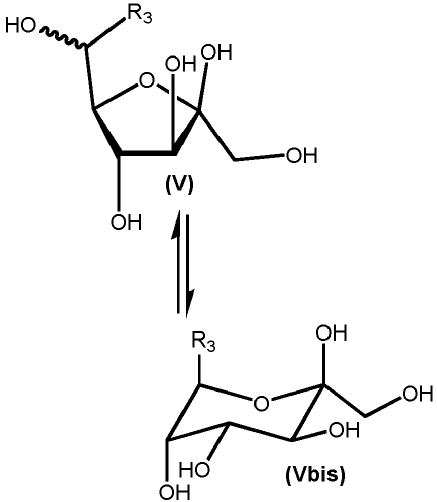 wherein R represents one chemical group selected among an ethyl, a n-propyl, an isopropyl, a n-butyl, an isobutyl, a cyclopentyl, a cyclohexyl, an allyl or-CH₂-Ph. Advantageously, Isolated compound according to claim 8 and being selected among the following diastereoisomers:

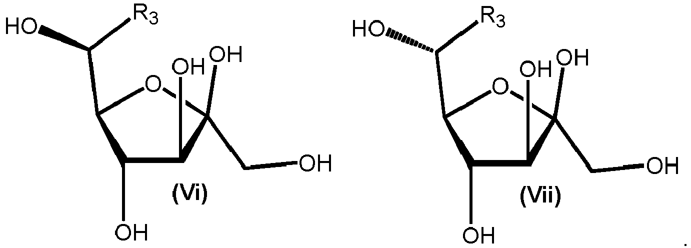

According to an another aspect, the invention relates to a biocidal composition, comprising an isolated compound according to the invention and/or salts thereof, and an agriculturally acceptable formulation auxiliary customary in crop protection.

Advantageously, the agriculturally acceptable formulation auxiliary customary in crop protection may be chosen from surfactants, preservatives, salts, pH adjustment agents, biostimulants, bioprotectors.

Advantageously, the biocidal composition according to the invention, may further comprise one or more further agrochemically active substances, optionally selected from the group consisting of biocides, and being advantageously selected among insecticides, acaricides, nematicides, herbicides, fungicides, safeners, fertilizers and/or further growth regulators.

The invention also relates to a method for controlling one or more harmful plants and/or for regulating the growth of one or more plants, comprising applying at least one of biocidally effective amount of the isolated compound according to the invention, and/or salts thereof, or the biocidal composition according to the invention to the plants, seeds of plants, soil in which or on which plants grow and/or an area under cultivation. For example, the plant may be Arabidopsis, vine, tomato plant, rapeseed.

The invention further relates to use of the compound according to the invention, and/or salts thereof, or the biocidal composition according to the invention as an antimicrobial agent, preferably as an antibacterial or antifungal agent.

### EXAMPLES

### Devices and protocols

NMR spectra were recorded on a Bruker Avance III 400 spectrometer operating at 400.13 MHz for 1H, equipped with a BBFO probe with a Z-gradient coil and a GREAT 1/10 gradient unit or on a Bruker Avance III operating at 500.13 MHz for 1H equipped with 1H and 13C cryo-probe. All experiments were carried out at 25 °C, and references for chemical shifts are external. Coupling constants J were calculated in hertz (Hz). Proton and carbon NMR peaks were unambiguously assigned by COSY (double quantum filtered with gradient pulse for selection), HSQC (gradient echo-anti echo selection and shape pulse), and HMBC (echo-anti echo gradient selection, magnitude mode) correlation experiments as well as TOCSY. Multiplicity are annotated as s (singlet), d (doublet), t (triplet), m (multiplet), or o (overlapping signals).

### Example 1: Synthetic method of 7dSh

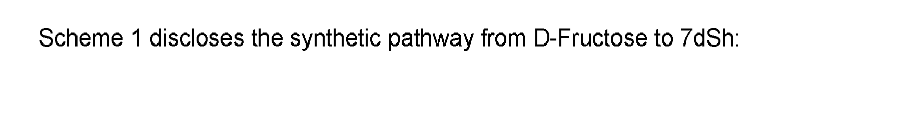

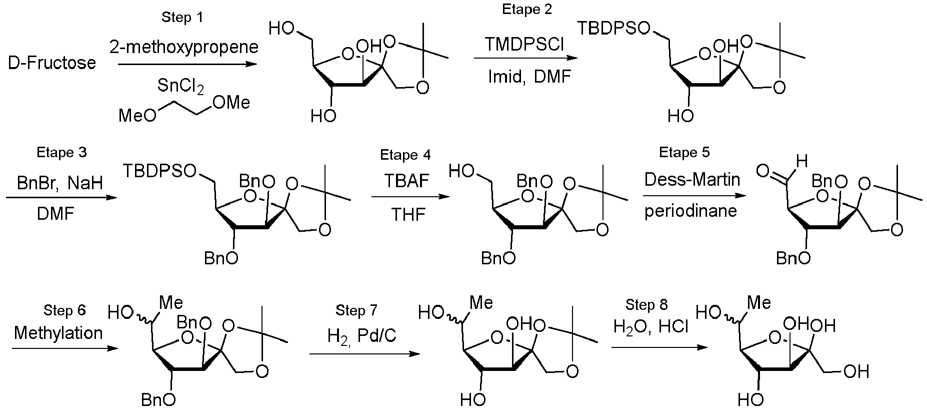

Steps 1-4 have already been performed in the literature, the aim of the present invention is to provide new synthetic route from step 5 to step 8; in one particular variant the synthetic pathway involve the alternative :

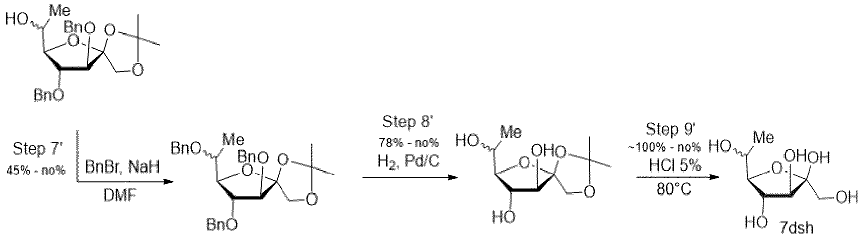

### (1) Synthesis of 1,2-O-isopropylidene-β-D-fructofuranose

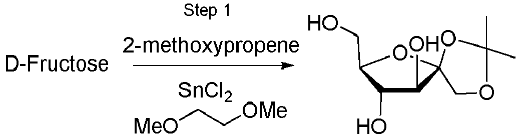

The preparation of 2-O-isopropylidene-β-D-fructofuranose was performed according to reference [5]:
A mixture of D-fructose (27 g, 0.15 mol, 1 eq.), 2-dimethoxypropene (50.2 mL, 0.53 mol, 3.5 eq.), and tin (II) chloride (160 mg, 0.84 mmol) suspended in 1,2-dimethoxyethane (1500 mL) was refluxed under inert atmosphere for 25 minutes. The reaction was terminated by the addition of pyridine (1.1 mL). Solvents were removed under reduced pressure. The crude was purified by column chromatography (CH₂Cl₂/MeOH 93:7 Rf = 0.21 then BuOH/NH₄OH/MeOH 9:0.5:0.5 Rf = 0.38) to yield 7.1 g of product as an yellow oil. NMR showed around 15 % of impurities, removed in the next step.

¹H NMR (400 MHz, CDCl₃) δ 4.25 (t, *J* = 7.4 Hz, 1H), 4.09 (2 dd, *J* = 9.4 Hz, 2H), 3.96 (d, *J* = 8.0 Hz, 1H), 3.92-3.84 (m, 1H), 3.72 (qd, 2H), 1.52 (s, 3H), 1.40 (s, 3H);

¹³C NMR (101 MHz, CDCl₃) δ 111.92, 108.66, 81.27, 77.69, 75.06, 70.71, 62.24, 26.93, 25.94.

### (2) Synthesis of 6-O-tert-Butyldiphenylsilyl-1,2-O-isopropylidene-β-D-fructofuranose

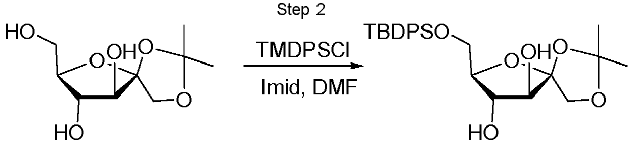

The preparation of 6-O-tert-Butyldiphenylsilyl-1,2-O-isopropylidene-β-D-fructofuranose was performed according to reference [6]:
To solution of 1,2-*O*-isopropylidene-*β*-D-fructofuranose (3.5 g, 15.9 mmol, 1 eq.) and imidazole (1.6 g, 23.5 mmol, 1.5 eq.) in dry DMF (40 mL) was added TBDPSCI (4.55 mL, 17.4 mmol, 1.1 eq.). The reaction was stirred overnight at room temperature. The solvent was removed under reduced pressure. The residue was extracted with AcOEt (30 mL) and water (10 mL). The organic layer was dried (MgSO₄), concentrated, and the resulting crude purified by column chromatography (toluene/AcOEt 8:2 Rf = 0.22) to obtain 1.46 g of the product as a white oil.

¹H NMR (400 MHz, CDCl₃) δ 7.78-7.67 (m, 4H), 7.45-7.38 (m, 6H), 4.18 (dd, *J* = 7.7, 6.7, 0.8 Hz, 1H), 4.08 (2 dd, 2H), 3.97 (dd, *J* = 7.9, 0.8 Hz, 1H), 3.88-3.84 (m, 1H), 3.84-3.80 (m, 2H), 1.48 (s, 3H), 1.41 (s, 3H), 1.10 (s, 9H);

¹³C NMR (101 MHz, CDCl₃) δ 135.7, 135.6, 133.4, 133.3, 129.9, 129.8, 127.8, 127.8, 111.4, 108.6, 80.7, 77.8, 77.0, 71.1, 64.7, 26.9, 26.6, 26.30, 19.4.

### (3) Synthesis of 3,4-Di-O-benzyl-6-O-tert-Butyldiphenylsilyl-1,2-O-isopropylidene-β-D-fructofuranose

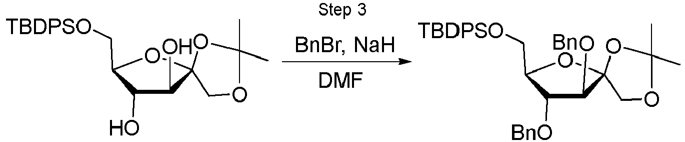

To a solution of 6-*O*-tert-butyldiphenylsilyl-1,2-*O*-isopropylidene-β-D-fructofuranose (2.25 g, 5 mmol, 1 eq.) in DMF (20 mL) at 0 °C was added benzyl bromide (1.8 mL, 14.7 mmol, 3 eq.) and a suspension of NaH (720 mg, 10.8 mmol, 2.2 eq.) in DMF (2.5 mL). The mixture was stirred at room temperature for 4h. MeOH (10 mL) was added to stop the reaction. Solvents were evaporated and the residue was extracted with AcOEt (30 mL) and water (10 mL). The organic layer was dried (MgSO₄) and concentrated. The crude was then purified by column chromatography (toluene to 50:1 toluene/AcOEt). The product was isolated as a white oil (2.25 g, 72 %).

¹H NMR (400 MHz, CDCl₃) δ 7.72-7.63 (m, 4H), 7.45-7.22 (m, 16H), 4.72 (s, 2H), 4.63 (2 dd, 2H,), 4.26 (dd, *J* = 6.3, 4.7 Hz, 1H), 4.07-4.01 (m, 1H), 3.97 (m, 3H), 3.80 (dd, *J* = 6.3, 3.7 Hz, 2H), 1.42 (s, 3H), 1.40 (s, 3H), 1.07 (s, 9H) ;

¹³C NMR (101 MHz, CDCl₃) δ 137.9-127.6, 111.5, 109.0, 84.5, 83.3, 81.7, 72.5, 72.3, 71.6, 65.6, 27.0, 26.5, 26.5, 19.4.

### (4) Synthesis of 3,4-Di-O-benzyl-1,2-O-isopropylidene-β-D-fructofuranose

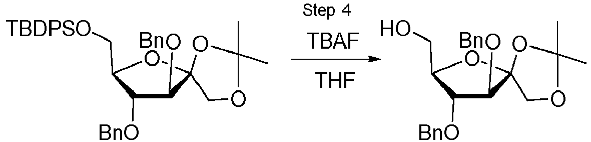

To a solution of 3,4-Di-O-benzyl-6-O-tert-butyldiphenylsilyl-1,2-O-isopropylidene-β-D-fructofuranose (900 mg, 1.4 mmol, 1 eq.) in THF (35 mL) was added TBAF (1 M in THF, 1.6 mL, 1.6 mmol, 1.1 eq.) at 0 °C. The reaction was stirred at room temperature for 3 h. The mixture was diluted in diethyl ether (30 mL) and washed with water (2×10 mL), dried (MgSO₄) and concentrated. The crude was purified by flash chromatography (Cyclohexane/AcOEt 9:1). The product was isolated as a white oil (400 mg, 71 %).

¹H NMR (400 MHz, CDCl₃) δ 7.40-7.29 (m, 10H), 4.76 (s, 2H), 4.66 (dd, 2H), 4.42 (dd, *J* = 7.0, 5.6 Hz, 1H), 4.09-3.98 (m, 4H), 3.67 (dd, 2H), 1.55 (s, 3H), 1.48 (s, 3H) ;

¹³C NMR (101 MHz, CDCl₃) δ 137.8-127.8, 111.7, 108.6, 83.2, 81.8, 81.8, 72.8, 72.3, 71.1, 63.5, 26.5, 26.1.

### (5) Synthesis of 3,4-Di-O-benzyl-1,2-O-isopropylidene-β-D-fructofuranuraldehyde

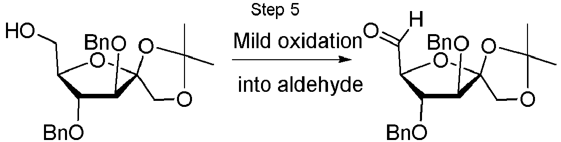

The mild oxidation can be performed according to the Dess Martin oxidation, or according to the Swern oxidation:
To a solution of DMSO (4.5 eq.) in dry dichloromethane (6 mL) was added chloride oxalyl (2.2 eq.) at -65 °C. The mixture was stirred for 40 min. The 3,4-di-*O*-benzyl-1,2-*O*-isopropylidene-*β*-*D*-fructofuranose (1 eq.) diluted into CH₂Cl₂ (4 mL) was added to the mixture and the reaction was stirred for 1 h, allowed to reach room temperature. Triethylamine (0.5 mL) was added to buffer the reaction at basic pH. The reaction was quenched with an excess of trimethylamine (10 mL). Solvents were evaporated and the product was extracted with dichloromethane (10 mL), washed with brine (3x15 mL) and pure water (3x15 mL). The organic phase was dried with Na₂SO₄ and concentrated, then diluted in chloroform to extract a white solid. A chromatographic column flash (eluent: cyclohexane/toluene/AcOEt/MeOH (3.5:5:0.75:0.75 - Rf ~ 0.28) gave a product as a light-yellowish oil (125 mg).

### NMR spectra:

-> ¹H NMR (400 MHz, CDCl₃), see figure 1; δ 9.80 (s, 1H, CHO), 7.47-7.13 (m, 10H, Ph), 4.73 (d, 1H, J= 11.4 Hz, CH₂Ph), 4.71 (d, 1H, J= 12.0 Hz, CH₂Ph), 4.67 (d, 1H, J= 12.1 Hz, CH₂Ph), 4.57 (d, 1H, J= 11.8 Hz, CH₂Ph), 4.36 (dd, 1H, J=3.7, 5.7 Hz), 4.28 (dd, 1H, J=1.1, 4.0 Hz), 4.14 (d, 1H, J=9.8 Hz), 4.01 (d, 1H, J=5.4 Hz, H-1), 3.99 (d, 1H, J=2.4 Hz, H-1'), 1.58 (s, 3H, CH₃), 1.46 (s, 3H, CH₃).
-> ¹³C NMR (101 MHz, CDCl₃), see figure 2; δ 202.44 (C=O), 137.16-127.62 (CH₂Ph), 112.03 (C(CH₃)₂), 109.95 (C-2), 85.45 (C-5), 83.03 (C-3), 81.41 (C-4), 72.11 (CH₂Ph), 72.02 (CH₂Ph), 70.28 (C-1), 26.36 (CH₃), 25.80(CH₃).

### (6) Synthesis of 3,4-di-O-benzyl-7-deoxy-1,2-O-isopropylidene-heptofuranose

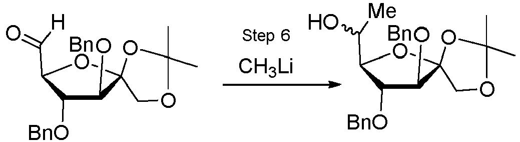

The preparation of 3,4-di-Q-benzyl-7 -deoxy-1,2-Q-isopropylidene-heptofuranose was performed according to reference [7]:
A solution of aldehyde (1 equiv.) in hexane (5 mL for 1 g) was added to a stirred solution of MeLi (2 equiv.) cooled at -70 °C. The mixture was allowed to warm at RT and stirred overnight. After washing with water, drying over MgSO₄ and removal of the solvent, the crude product was purified by flash-chromatography (Cyclohexane/AcOEt).

### NMR spectra:

-> ¹H NMR (400 MHz, CDCl₃), δ 7.35-7.21 (m, 10H, Ph), 4.75 (d, 1H, J= 11.4 Hz, CH₂Ph), 4.72 (d, 1H, J=12.0 Hz, H-1'), 4.64 (d, 1H, H-1), 4.06 (d, 1H, J=12.0 Hz, CH₂Ph), 4.02 (d, 1H, J= 11.8 Hz, CH₂Ph), 4.42 (dd, 1H, J=5.6, 6.7 Hz, H-4), 4.07 (dd, 1H, J=1.1, 4.0 Hz, H-3), 3.85 (dd, 1H, J=3.2, 5.6 Hz, H-5), 3.98-3.93 (m, 1H, H-6), 1.50 (s, 3H, CCH₃), 1.45 (s, 3H, CCH₃), 1.15 (d, 1H, J=6.4 Hz, C*H*₃CH).
-> ¹³C NMR (101 MHz, CDCl₃): δ 137.16-127.62 (CH₂Ph), 111.80 (C-2), 108.85 (C(CH₃)₂), 84.86 (C-5), 84.86 (C-5), 84.76 (C-3), 81.05 (C-4), 72.26 (CH₂Ph), 71.63 (CH₂Ph), 72.53 (C-1), 67.50 (C-6), 26.38 (C(CH₃)₂), 26.13 (C(CH₃)₂), 17.76 (CH₃CH).

### (7) Synthesis of 7-deoxy-1,2-O-isopropylidene-heptofuranose

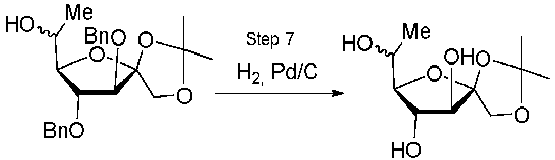

The preparation of 7-deoxy-1,2-O-isopropylidene-heptofuranose was performed according to reference [8]:
A solution of dibenzylated intermediate (1 equiv.) 1.77 g, 3.73 mmol) in AcOH/AcOEt (1:1, 1 mL for 1 g) added of palladium(ii) acetate (0.1 equiv.) was stirred for 72 h under 1 atm of hydrogen. After completion of the deprotection, the solvents were removed by co-evaporation with MeOH (5×50 mL) and a chromatographic purification (CH₂Cl₂/MeOH) gave the desired product.

### (8) Synthesis of 7-deoxy-heptose

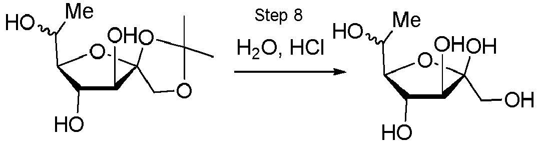

The preparation of 7-deoxy-heptose was performed according to reference [9]:
To a solution of the isopropylydene derivative (1 equiv.) in DCM (5 mL for 100 mg) were added 1 drop of water and TFA (5 equiv.). The mixture was stirred for 15 h at room temperature. Then the reaction was quenched by adding triethylamine, and the solvent was removed under reduced pressure. The resulting residue was chromatographically purified (cyclohexane/AcOEt) to afford the desired product.

### Alternative route:

### (9) Synthesis of 3,4,7-tri-O-benzyl-1,2-O-isopropylidene-heptofuranose

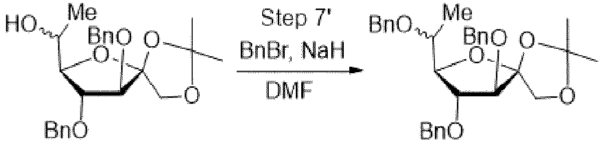

Benzylation step was performed as described for step 3 starting from 3,4-di-O-benzyl-1,2-O-isopropylidene-heptofuranose (4.15 g, 0.01 mol), benzyl bromide (2 mL, 0.017 mol), a 60% powder of NaH (860 mg, 0.014 mmol), in DMF (28 mL). After purification by flash chromatography eluting with (AcOEt/Cyclohexane, 50:50), the desired product was isolated in 45% yield (1.98 g).

### NMR spectra:

-> ¹H NMR (400 MHz, CDCl₃), δ 7.47-7.21 (m, 15H, Ph), 4.71 (d, 1H, J= 11.2 Hz, CH₂Ph), 4.71 (s, 2H, CH₂Ph), 4.71 (d, 1H, J= 10.4 Hz, CH₂Ph), 4.66 (d, 1H, J= 12.0 Hz, CH₂Ph), 4.54 (d, 1H, J=12.0 Hz, CH₂Ph), 4.50 (d, 1H, J= 11.2 Hz, CH₂Ph), 4.26 (dd, 1H, J=4.0, 6.0 Hz, H-4), 4.09 (d, 1H, J=9.2 Hz, H-1), 4.03 (d, 1H, J=6.9 Hz, H-3), 3.98 (d, 1H, H-1'), 3.81 (dd, 1H, J=4.0, 8.0 Hz, H-5), 3.76-3.71 (m, AH, H-6), 1.54 (s, 3H, CH₃), 1.50 (s, 3H, CH₃), 1.34 (d, 1H, J=6.0 Hz, C*H*₃CH).
-> ¹³C NMR (101 MHz, CDCl₃): δ 137.58-127.47 (CH₂Ph), 111.53 (C(CH₃)₂), 109.12 (C-2), 84.95 (C-4), 84.56 (C-5), 83.75 (C-3), 76.33 (C-6), 72.21 (CH₂Ph), 72.10 (CH₂Ph), 71.59 (C-1), 71.17 (CH₂Ph), 26.40 (C(CH₃)₂), 26.36 (C(CH₃)₂), 16.18 (CH₃CH).

Steps 8' and 9' where performed according to protocols described above in point (7) and (8) to yield expected 7-deoxy-heptose in 100% yield (last step), while the step 8' provided 7-deoxy-*1,2*-O-isopropylidene-heptofuranose in 78% yield.

### List of references

[1] Defarge, N. ; Spiroux de Vendômois, J. ; Séralini, G. E. "Toxicity of formulants and heavy metals in glyphosate-based herbicides and other pesticides." Toxicology Reports, 2018, 5, 156-163.
[2] Mesnage, R. ; Benbrook, C. ; Antoniou, M. N. "Insight into the confusion over surfactant co-formulants in glyphosate-based herbicides." Food and Chemical Toxicology, 2019, 128, 137-145.
[3] Brilisauer, K., Rapp, J., Rath, P. et al. Cyanobacterial antimetabolite 7-deoxy-sedoheptulose blocks the shikimate pathway to inhibit the growth of prototrophic organisms. Nat Commun 10, 545, 2019. https://doi.org/10.1038/s41467-019-08476-8.
[4] Greene's Protective Groups in Organic Synthesis, Print ISBN:9781118057483, Online ISBN:9781118905074, DOI10.1002/9781118905074
[5]: Chittenden, G. F. "1,2-O-Isopropilydene-β-D-fructofuranose. A new acetal of D-fructose.", J. Chem. Soc., Chem. Commun. 1980, 641, 882-883 ;
[6]: Rubio, E. M. et al. "Spacer-Mediated Synthesis of Contra-Thermodynamic Spiroacetals: Stereoselective Synthesis of C 2 -Symmetric Difructose Dianhydrides." J. Org. Chem. 2006, 71, 2257-2266 ;
[7]: Nozaki N. et al. "Asymmetric synthesis by means of (-)-sparteine modified organometallic reagents" Tetrahedron 1971, 27, 905-913;
[8]: Euzen R et al. "A Chemoenzymatic Approach for the Synthesis of Unnatural Disaccharides Containing D-Galacto- or D-Fucofuranosides" Eur. J. Org. Chem. 2005, 4860-4869 ;
[9]: Favreau B. et al. "Synthesis of an Exhaustive Library of Naturally Occurring Galf-Manp and Galp-Manp Disaccharides. Toward Fingerprinting According to Ring Size by Advanced Mass Spectrometry-Based IM-MS and IRMPD J. Org. Chem. 2021 https://doi.org/10.1021/acs.joc.1c00250

## Claims

1. Process for the preparation of a biocidal compound comprising the steps of:
a) oxidizing primary alcohol group of intermediate (I) wherein R₂ represents a suitable secondary alcohol protective group, such as a benzyl protective group to form intermediate (II) according to the following scheme : 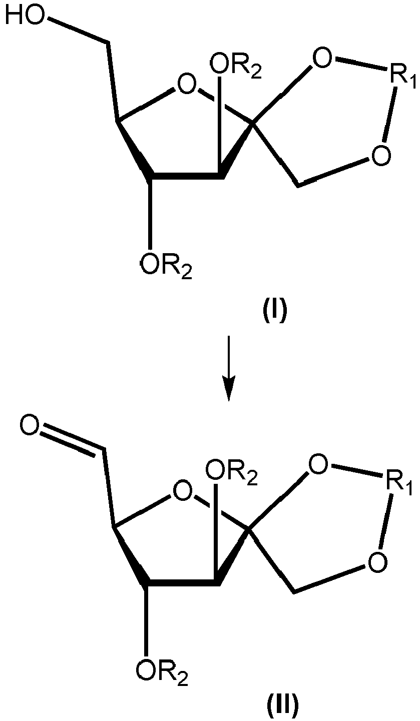 wherein R₂ represents a suitable secondary alcohol protective group, such as a benzyl protective group; and wherein R₁ is selected from saturated and unsaturated linear or branched aliphatic groups, and is preferably selected amongs: alkyl, alkenyl, alkynyl, eventually bearing an aryl group;
b) alkylating the aldehyde moiety of intermediate (II) under suitable conditions to form intermediate (III) : 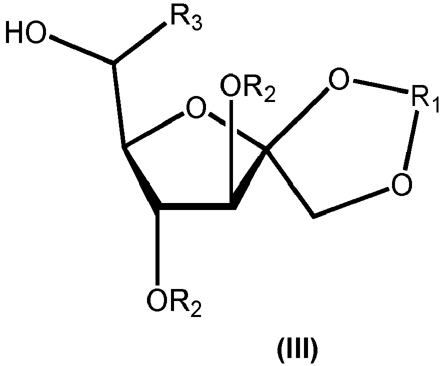 wherein R₃ represents a cyclic or acyclic, substituted or unsubstituted, linear or branched C₁₋₁₂alkyl, C₁₋₁₂alkoxyl, C₁₋₁₀alkyl-CH=CH₂ or -CH₂-Ar group ; and
c) removing the R₂ protective groups to yield a compound having the structure (IV): 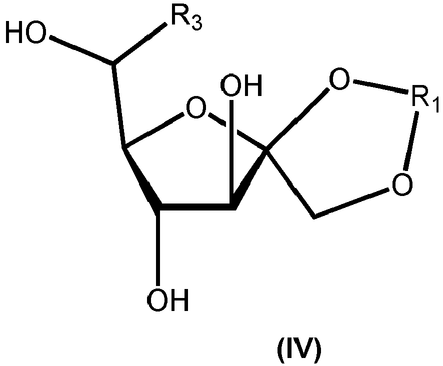
d) removing the R₁ protective groups to yield a compound having the structure (V):

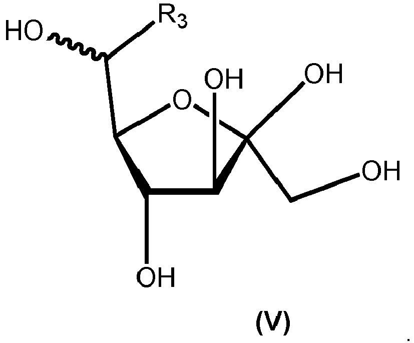

2. Process according to claim 1, comprising a supplementary! step e) performed before step c) and being according to the following:
e) protecting the primary alcool group of intermediate (III) under suitable conditions to form intermediate (VI) : 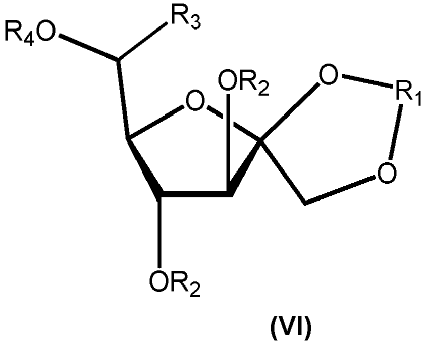 wherein R₄ represents a suitable primary alcohol protective group, such as a benzyl protective group, step c) consisting in deprotecting R₄ together with R₂ protecting group.

3. Process according to claim 2, wherein R₂ and R₄ are benzyl protecting groups and step c) consists of deprotecting the benzyl protectives groups to provide free -OH groups to form intermediate IV.

4. Process according to any of claims 1 to 3, wherein R₁ represents one of the following groups selected among:

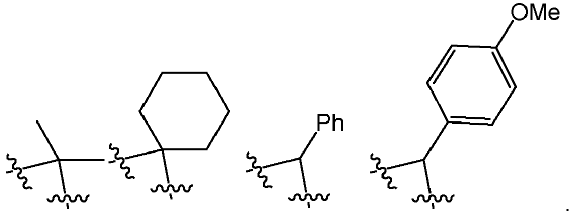

5. Process according to any of claims 1 to 4, wherein step a) is a mild oxidation step, preferably a Dess Martin oxidation step, which is carried out by reacting intermediate (I) with Dess Martin periodinane.

6. Process according to any of claims 1 to 5, wherein step b) is carried out by reacting intermediate (II) with R₃Li or (R₃)₂ZnTi(OiPr)₄; wherein R₃ represents a cyclic or acyclic, substituted or unsubstituted, linear or branched C₁₋₁₂alkyl, C₁₋₁₂alkoxyl, C₁₋₁₀alkyl-CH=CH₂ or -CH₂-Ph group.

7. Process according to any of claims 1 to 6, wherein R₃ represents methyl, ethyl, isopropyl, n-butyl, isobutyl, cyclopentyl, cyclohexyl, allyl or-CH₂-Ph; preferably methyl.

8. Isolated compound obtained by the method according to anyone of claims 1 to 7, and having one of the following formula (V) and (Vbis) : 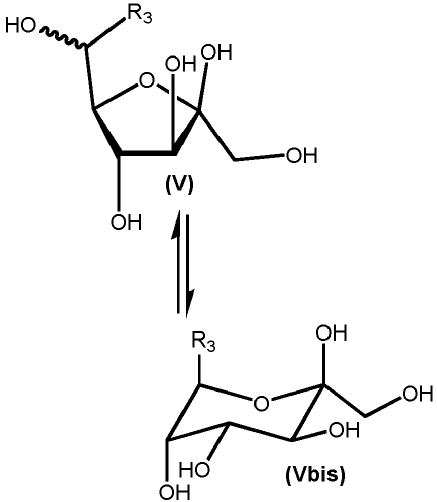 wherein R₃ represents one chemical group selected among an ethyl, a n-propyl, an isopropyl, a n-butyl, an isobutyl, a cyclopentyl, a cyclohexyl, an allyl or -CH₂-Ph.

9. Isolated compound according to claim 8 and being selected among the following diastereoisomers:

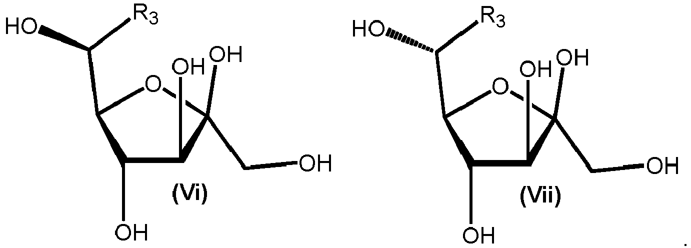

10. A biocidal composition, comprising an isolated compound according to any one of claims 8 or 9 and/or salts thereof, and an agriculturally acceptable formulation auxiliary customary in crop protection.

11. Biocidal composition according to claim 10, further comprising one or more further agrochemically active substances, optionally selected from the group consisting of insecticides, acaricides, nematicides, herbicides, fungicides, safeners, fertilizers and/or further growth regulators.

12. A herbicidal method for controlling one or more harmful plants and/or for regulating the growth of one or more plants, comprising applying at least one of biocidally effective amount of the compound according to any one of claims 8 or 9, and/or salts thereof, or the biocidal composition according to any one of claims 13 to 14 to the plants, seeds of plants, soil in which or on which plants grow and/or an area under cultivation.

13. Use of the biocidal composition according to any one of claims 8 or 9 and/or salts thereof, and/or salts thereof, or the biocidal composition according to any one of claims 10 to 11 as an antimicrobial agent, preferably as an antibacterial or antifungal agent.
